Europäisches Patentamt

(19) European Patent Office

Office européen des brevets

(11) Publication number : **0 441 750 A1**

# (12) EUROPEAN PATENT APPLICATION

(21) Application number : **91810071.0**

(22) Date of filing : **01.02.91**

(51) Int. Cl.⁵ : **A01K 51/00**

(30) Priority : **05.02.90 GB 9002505**

(43) Date of publication of application :
**14.08.91 Bulletin 91/33**

(84) Designated Contracting States :
**AT BE CH DE DK ES FR GB GR IT LI LU NL SE**

(71) Applicant : **SANDOZ LTD.**
**Lichtstrasse 35**
**CH-4002 Basel (CH)**
**BE CH DK ES FR GB GR IT LI LU NL SE**
Applicant : **SANDOZ-PATENT-GMBH**
**Humboldtstrasse 3**
**W-7850 Lörrach (DE)**
**DE**
Applicant : **SANDOZ ERFINDUNGEN**
**VERWALTUNGSGESELLSCHAFT M.B.H.**
**Brunner Strasse 59**
**A-1235 Vienna (AT)**
**AT**

(72) Inventor : **Entner, Reinhard**
**A-6250 Breitenbach 251 (AT)**

(54) **Improvements in or relating to mechanical devices.**

㊗   A device for applying an active ingredient to an insect comprising a sluice containing an active ingredient and having at least one aperture adapted to allow passage of the insect therethrough is described.

··FIG.4··

EP 0 441 750 A1

## IMPROVEMENTS IN OR RELATING TO MECHANICAL DEVICES

This application concerns a device and method for applying an active ingredient to an insect. While there are various situations where it is desired to apply an active ingredient to an insect, this invention primarily concerns itself with protecting an insect against attack by ectoparasites such as other insects or mites through the use of a sluice adapted to release a controlled effective amount of an active ingredient.

Various devices for applying a compound to an insect have been previously proposed. One such device consists of a long narrow strip that releases a controlled amount of a compound and which is placed at or near the locus of the insect to be treated. These strips have been particularly useful for protecting bees against attack from mites, particularly the Varroa jacobsoni mite. Such strips, commercially known by the name Apistan^R (available from Zoecon Corp., Dallas, TX), contain the compound fluvalinate ((RS)-$\alpha$-cyano-3-phenoxybenzyl N-(2-chloro-$\alpha,\alpha,\alpha$-trifluoro-p-tolyl)-D-valinate), which is a potent insecticide and acaricide. These strips may be placed at or near the entrance of a beehive. The bees will then typically crawl across the strips before or after entrance to or exit from the hive, and thereby be treated with fluvalinate. Alternatively, the strips may be placed at or near the brooding region of a beehive. Treatment takes place during the brooding season, and typically after the end of the honey harvest.

Such strips may not be effective for treating bees in all situations. When placed at or near the entrance to the beehive, their effectiveness may be hindered since the bees can often fly over the strips without having to crawl across them. When such strips are placed at or near the brooding region of the hive, they are sometimes forgotten and left in place during the honey harvest season, in which case the honey may be contaminated with fluvalinate. In addition, the beehive must be disturbed, since it must be taken apart to insert the strips in the brooding region. Moreover, strips that are placed inside the hive are sometimes covered with wax and thereby rendered ineffective.

The foregoing disadvantages are avoided by the device of this invention, which is illustrated in the drawings and described below.

Figure 1 illustrates a front-view of a sluice 1 according to this invention.

Figure 2 illustrates a conventional bee hive 4.

Figure 3 illustrates a beehive according to this invention.

Figure 4 illustrates an embodiment of the sluice of this invention in which sluice 1 is mounted on a hollow support 8 which is adapted to be removably inserted in the beehive of Figure 3.

Figure 5 is a photograph of the sluice of Figure 4 is partially inserted in the beehive of Figure 3.

Figure 6 is a photograph of the sluice of Figure 4 fully inserted in the bee hive of Figure 3.

Figures 7, 8 and 9 illustrate further configuations for mounting the sluice on a support.

This invention particularly concerns a device for applying an active ingredient to an insect, comprising a sluice capable of releasing a controlled effective amount of said active ingredient and having at least one aperture adapted to allow passage of the insect therethrough.

Active ingredient, as used herein, refers to any type of active ingredient that is suitable for treating an insect that is diseased, infested with a parasite or otherwise in need of treatment. The active ingredients particularly contemplated by this invention are those which are effective in controlling parasitosis in honey bees.

Parasitosis in honey bees is caused by such parasites as Varroa jacobsoni, Acarapis woodi, Tropilaelaps clareae, Tropilaelaps koenigerum, Braula coeca, Galleria mellonella and Achroia grisella. At present, the parasite of utmost concern is Varroa jacobsoni, as it is the most prevalent and is known to harm the bee at various stages of its development in addition to the adult bee. Accordingly, this invention is especially concerned with active ingredients that are effective in controlling Varroa jacobsoni on honey bees.

Suitable active ingredients for controll of Varroa jacobsoni on honey bees include the synthetic pyrethroids, fluvalinate (available from Zoecon Corp.) and flumethrin (available from Bayer AG).

Other acaricides and/or insecticides contemplated by this invention include amitraz (available from Schering AG) ; organophosphate compounds, e.g., coumaphos (available from Bayer AG) ; fungicidal agests for the control of various bacterial or fungal disorders, e.g. menthol or inilconazole (available from Janssen) ; as well as insect growth regulators and feeding enhancement agents.

Insects, as used herein, refer particularly to flying insects and insects which reside in nests, including ants, bees, wasps, and hornets. The device of this invention is particularly suitable for providing convenient, environmentally acceptable controlled release delivery of active ingredients adapted to treat bees.

Sluice, as used herein, refers to any three dimensional, solid figure. A particularly suitable sluice is plate-like, that is, it is a substantially planar figure whose breadth is greater than its thickness. Thus, a sluice according to this invention may take on any shape, e.g., it may be circular, rectangular, or irregularly shaped, depending upon where it is ultimately used. A particularly preferred sluice is in the form of a substantially rectangular plate or strip.

The sluice may be made from any suitable material, e.g., wood, cardboard, rubber felt, metal, ceramic materials or plastic. Plastics include polyacrylate, polymethacrylate, epoxide, polyurethane, polyester, and polyamide, although such plastics as polyvinylchloride, polyethylene, polypropylene, and butadienepolystyrol are particularly preferred, as they are especially durable and can easily be shaped and formed.

Aperture adapted to allow passage of the insect, as used herein, refers to an opening in the sluice which allows an insect to pass from one side of the sluice to the other side. Apertures may be of any shape, e.g., square, rectangular, or round, although they are most conveniently round.

The size of the aperture is suitably chosen such that insects are forced to crawl through one of the apertures. The insects thereby come into intimate and thorough contact with the active ingredient. In the case where the device of this invention is placed near an insect nest, all insects are eventually treated with the active ingredient, either directly by the sluice, or indirectly by contact with insects that have already been treated. Thus, the device of this invention provides a very effective means for applying an active ingredient to an insect colony.

In the case where bees are to be treated, the apertures will typically be round and range from about 5 to 10 mm in diameter. The number of apertures is not critical, although preferably there are a sufficient number to allow the bees easy access to the hive. The sluice will generally have from 1 to 100 apertures, more typically 5 to 50 apertures, although the exact number of apertures in the sluice will depend on various factors, including the type insect, the number of insects to be treated, and the size of the sluice.

Sluice containing an active ingredient, as used herein, refers to an active ingredient that lies at least on the surface of the sluice, and thus, the active ingredient is applied to or treats an insect when the insect comes into contact with the sluice. The member, therefore, may be impregnated with the active ingredient, coated with the active ingredient, or alternatively, the active ingredient may be an integral ingredient in the composition that forms the sluice.

In the case where the sluice is coated with the active ingredient, the coating may comprise, in addition to the active ingredient, an adherent polymer and where appropriate additional fillers. Suitable polymers are the surface coating raw materials of the paint industry and, for example, cellulose derivatives, acrylates and methacrylates.

Where the coating comprises an adherent polymer in addition to the active ingredient, the active ingredient generally will constitute up to 30% by weight of the coating, preferably from 0.001-20% by weight, depending upon the efficacy of the active ingredient.

Examples of fillers to be used in a coating are kaolin, calcium cabonate, silicates, and bentonites.

In a preferred embodiment, the active ingredient is an integral ingredient in the composition that forms the sluice. The active ingredient suitably comprises up to 30% by weight of the composition that forms the sluice, more typically from 0.001-20% by weight, depending upon the efficacy of the active ingredient. In this embodiment, the active ingredient will be combined with a plastic that permits adequate migration of the active compound to the surface of the sluice, for example, one of the above-mentioned plastics. The composition of this embodiment may further contain plasticizers, stabilizers, lubricants, fillers and coloring material.

Suitable plasticizers are those which are customarily used with the aforementioned plastics. Examples of such plasticizers are esters of phosphoric acid such as tricresyl phosphate, esters of phthalic acid such as dimethyl phthalate and dioctyl phthalate and esters of adipic acid such as diisobutyl adipate. Other plasticizers include esters of azelaic acid, maleic acid, ricinoleic acid, myristic acid, palmitic acid, as well as complex linear polyesters, and epoxidized soybean oils. The amount of plasticizer will typically vary from 10-50% by weight of the composition.

Suitable stabilizers are antioxidants and agents which protect the shaped article from ultraviolet radiation and disintegration during processing. Suitable lubricants are stearates, stearic acid and low molecular-weight polyethylene. Suitable fillers are as mentioned above. These components may be present up to 20% by weight of the composition

The sluice may be processed according to known methods for the production of shaped articles. Suitable processes for forming the sluice from one or more plastics include casting, pressing, injection molding, spreading, extrusion calendering and rolling, and are selected according to the individual properties of the plastic(s).

In one embodiment, the sluice is prepared according to the following procedure. Components are given in parts by weight.

Example 1

Fifty parts of polyvinyl chloride are placed in a high-speed mixer (Henschel mixer, 1800 rpm) and 35 parts of dioctyl phthalate and 4.75 parts of epoxidized soybean oil are added and mixed with the polyvinyl chloride until all of the liquids are absorbed and the resulting mixture is free-flowing. Ten parts of fluvalinate and 0.25 parts of stearic acid are added and mixed briefly. The mixture is then dropped from the mixer and extruded (Killion extruder) to provide a rectangular strip that is approximately 25 cm long, 3.2 cm wide and 0.09 cm thick, which is allowed to dry at room temperature. Subsequently, two rows of twelve holes, having a diameter of 7.5 mm and being spaced approximately one cm apart are bored into the strip.

The polyvinyl chlorde employed above is a high molecular weight resin, specific gravity 1.40, relative viscosity (avg.) 2.50 as measure in cyclohexane at 25°C, volatile content 0.5%, bulk density (avg.), gm/cc 0.53.

The epoxidized soybean oil is a clear, pale yellow liquid of molecular weight of about 420.

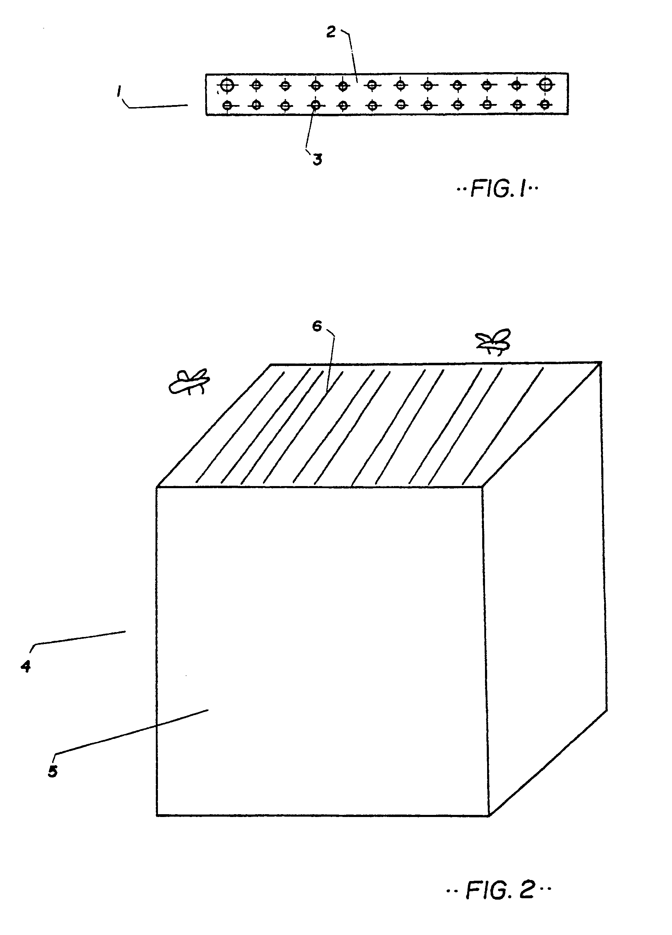

The sluice as prepared according to Example 1 is depicted in Figure 1. In this embodiment, sluice 1 is in the shape of a strip 2 and contains 24 equally spaced, round apertures 3.

The device of this invention has particular advantages when used to apply an active ingredient to bees. Treatment takes place at the point where the bees enter the beehive. There is no need to enter the brood comb to treat the bees, and thus, no need to disturb the usual development and living processes of the bees. Moreover, treatment can be easily discontinued at appropriate times, e.g., during pollen collection, simply by removing the sluice from the beehive.

An example of a conventionally known beehive is illustrated in Figure 2. Such a beehive consists of a bottom section (not shown), sides 5 and a top section 6. Typically the top is not a solid body, but rather contains openings which allow the bees to enter the beehive and access the bee colonies therein. The beehive is typically made from materials such as metal, wood or plastic.

Of course, conventional beehives may take on various configurations, for example, the sides may contain openings to allow passage of the bees whereas the top is a solid body. These variations are well within the skill of the art.

In the invention of this application, however, a conventional beehive is adapted so as to accommodate a sluice whereby the sluice constitutes the only access point through which the bees can enter the beehive. By accommodate it is meant that the beehive contains an opening at which or within which the sluice may be placed. In order that the bees must pass through the sluice to enter or exit the beehive, the beehive of this invention either contains no additional openings in its top and/or sides, or otherwise, any openings in the top and/or sides of the beehive must be made small enough to prevent passage of the bees. Preferably, the beehive of this invention contains such additional openings so as to improve ventilation of the hive. Furthermore, the beehive may be adapted to accommodate more than one sluice.

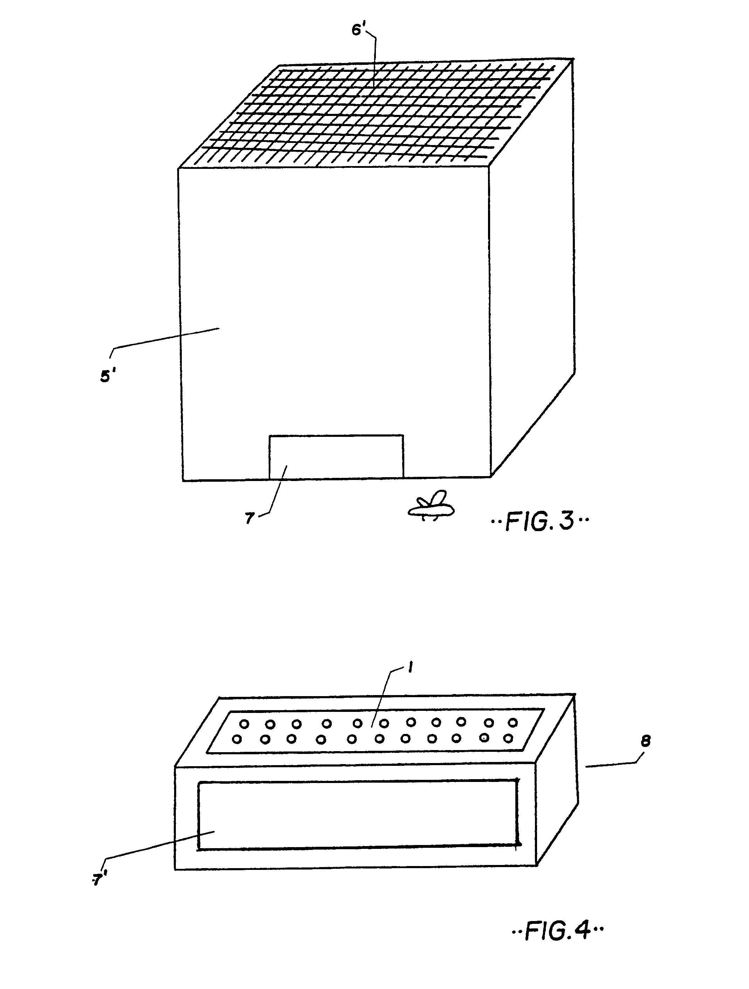

One embodiment of a beehive to be used in conjuction with the sluice of this application is depicted in Figure 3. In this beehive, the openings in top 6' are made smaller to prevent passage of bees therethrough. An opening 7 is made in side 5' at or within which the sluice can be positioned.

In a preferred embodiment of the device of this invention, the sluice is mounted on a support, which is adapted to support the sluice in a fixed position. The support may be so configured to guide the insect to the apertures of the sluice. The support may be comprised of a single member, e.g., it may form a frame around the sluice. Alternatively, the support may comprise several members, e.g. a top member, side members, and a bottom member, which attach to a top, side, and bottom portion, respectively, of the sluice, and which effectively support the sluice in a fixed position and guide insects towards an aperture of the sluice. Such supports may be made from any suitable material, e.g., metal, plastic, or wood, and may be the same or different from the material of the sluice.

Figure 4 depicts an embodiment of the invention of this application wherein sluice 1 is mounted on a hollow support 8. Support 8 is box shaped and is adapted to be removably inserted into opening 7 of the beehive of Figure 3. As indicated in Figure 4, one face of support 8 forms a frame around sluice 1 and an opening 7' is formed in a second face of support 8 to allow bees to enter the support and subsequently pass through one of the apertures of the sluice in order to gain access to the beehive.

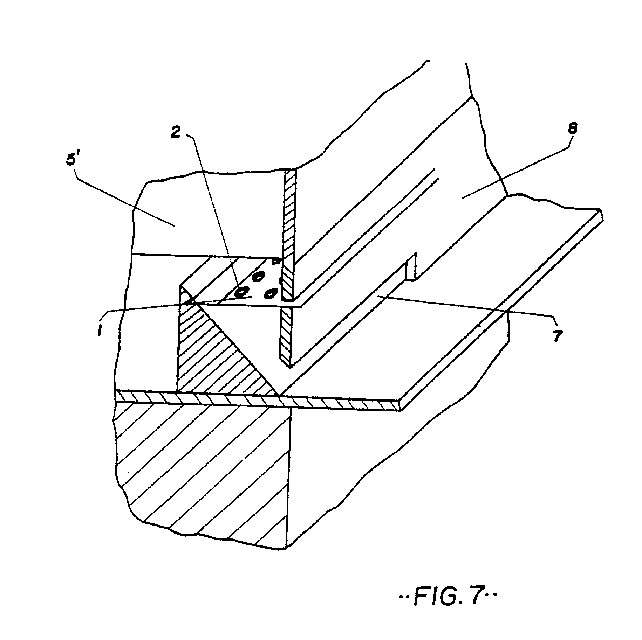

While the support in this embodiment is preferably box-shaped, it can of course take on other geometric shapes as desired, such as depicted in Figures 7, 8 and 9.

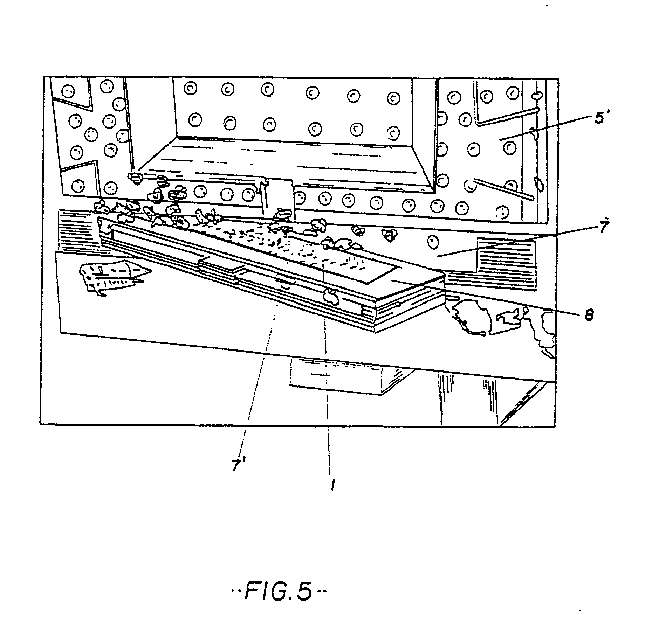

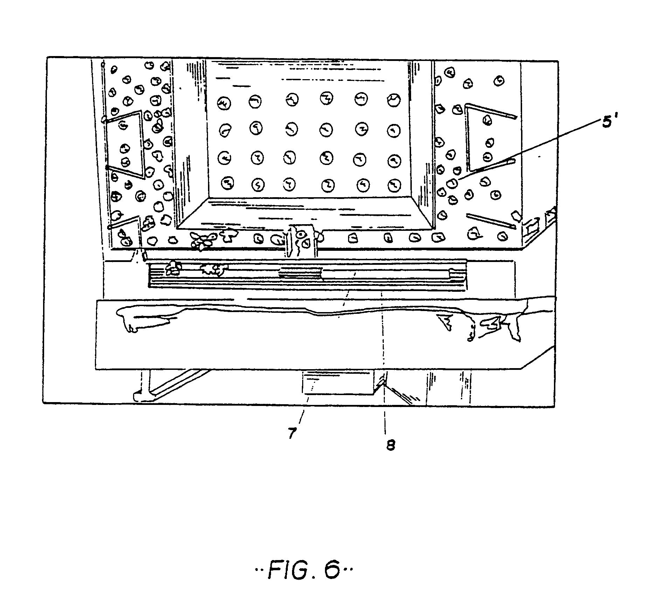

Figures 5 and 6 illustrate how the sluice and support of Figure 4 are used together with the beehive of Figure 3. As indicated in these figures, sluice 1 and support 8 are conveniently inserted into opening 7 of the beehive. Once inserted, sluice 1 represents the only access point for bees into and out of the beehive. At times when treatment of the bees is to be discontinued, e.g., during pollen collection, the sluice and support are simply removed from the opening of the behive.

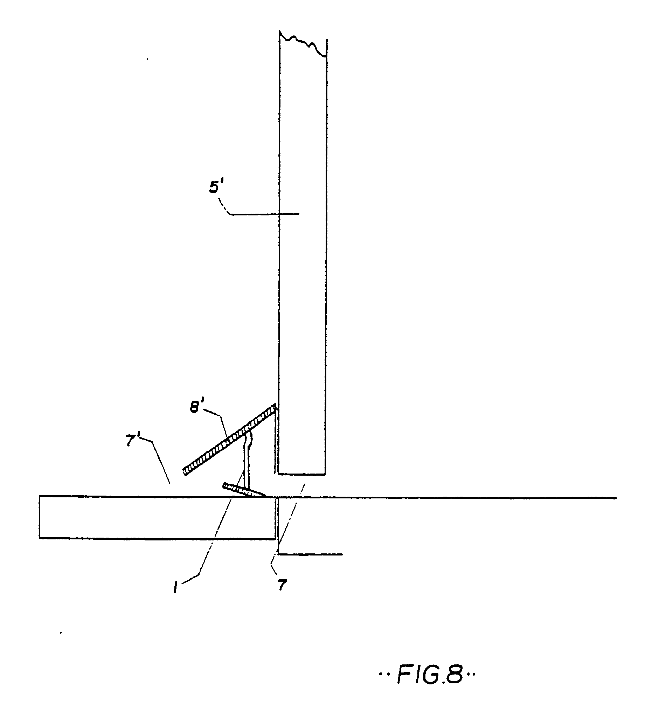

Figure 8 illustrates a cut-away view of another embodiment of the device of this invention, wherein sluice 1 is supported vertically by a support 8' having opening 7', and is placed at opening 7 of the beehive but is not inserted within the opening as in the embodiment of Figure 4.

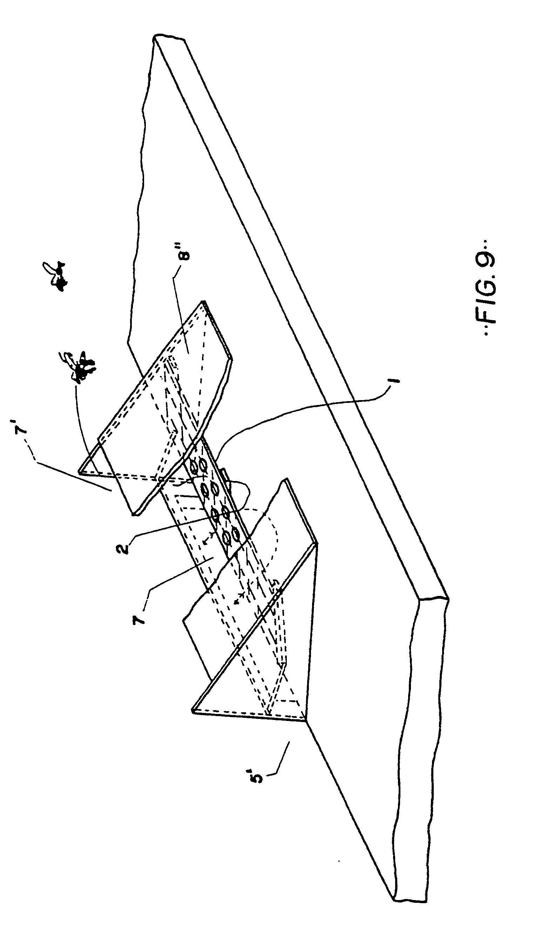

Figure 9 illustrates a further embodiment in which sluice 1 is positioned at an angle with respect to the horizontal by a support 8". As with the embodiment of Figure 8, the embodiment is place at the opening of beehive 7 but is not inserted within the opening.

## Claims

1. A device for applying an active ingredient to an insect comprising a sluice capable of releasing a controlled amount of an active ingredient and having at least one aperture adapted to allow passage of the insect therethrough.

2. A device according to claim 1 wherein said apertures are circular.

3. A device according to claims 1 or 2 wherein said apertures are from about 5 to 10 mm in diameter.

4. A device according to claims 1 to 3 wherein said sluice is mounted on a support, said support being adapted to support said sluice in a fixed position.

5. A device according to claims 1 to 4 wherein the active ingredient is effective against parasitosis in honey bees.

6. A device according to claims 1 to 5 wherein the active ingredient is effective against Varroa jacobsoni on honey bees.

7. A device according to claims 1 to 6 wherein the active ingredient is a selected from flumethrin and fluvalinate.

8. A device according to claims 1 to 7 wherein said active ingredient is fluvalinate.

9. A device according to claims 1 to 8 where said apertures are adapted to allow passage of bees.

10. A device according to claims 1 to 9 wherein the sluice is in the form of a substantially rectangular strip.

11. A device for applying an active ingredient to a bee comprising a beehive having a sluice as defined in claims 1-10 whereby bees must pass through said sluice in order to enter and exit the beehive.

·· FIG. I ··

·· FIG. 2 ··

6'

5'

7

··FIG. 3··

1

8

7'

··FIG.4··

··FIG.5··

··FIG. 6··

··FIG. 7··

·· FIG.8 ··

FIG. 9

**European Patent Office** · **EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 608 369 (SCHIMMENTI) <br> * the whole document * | 1,5,6,11 | A01K51/00 |
| X | US-A-4 349 981 (SHERMAN) <br> * the whole document * | 1 | |
| P,X | US-A-4 970 822 (SHERMAN) <br> * abstract; figures 1-3 * | 1,10 | |
| A | EP-A-224 697 (BAYER AG) <br> * page 1, line 15 - page 5, column 14; claims 1-3 * | 5-8 | |
| A | EP-A-211 207 (AMERICAN CYANAMID COMPANY) <br> * page 7; claim 8 * | 7,8 | |

TECHNICAL FIELDS SEARCHED (Int. Cl.5)

A01K
A01M

The present search report has been drawn up for all claims

| Place of search | Date of completion of the search | Examiner |
|---|---|---|
| THE HAGUE | 28 MAY 1991 | VON ARX V.U. |